# EUROPEAN PATENT APPLICATION

(11) **EP 4 306 513 A1**
(43) Date of publication of application: **17.01.2024**
(21) Application number: 22788465.7
(22) Date of filing: 14.04.2022
(51) Int. Cl.: C07D 231/56, C07C 45/86, C07C 45/61, C07C 45/81, C07C 49/563, C07C 49/333

(54) **METHOD FOR PREPARING INTERMEDIATE FOR SYNTHESIS OF SPHINGOSINE-1-PHOSPHATE RECEPTOR AGONIST**

(30) Priority: 14.04.2021 KR 20210048765
(71) Applicant: Lg Chem, Ltd., Seoul 07336 (KR)
(72) Inventor: KIM, Sung Wook, Daejeon 34122 (KR); KIM, Ki Dae, Daejeon 34122 (KR); LEE, Soo Min, Daejeon 34122 (KR)
(74) Representative: Hoffmann Eitle
(86) International application number: PCT/KR2022/005398
(87) International publication number: WO 2022/220610

(57) **Abstract**

The present invention relates to a novel method for producing a compound of chemical formula 4, which is disclosed in the present specification and can be effectively used for the synthesis of a sphingosine-1-phosphate receptor agonist.

## Description

### TECHNICAL FIELD

### Cross-reference to Related Applications

The present application claims the benefit of priority based on Korean Patent Application No. 10-2021-0048765, filed on April 14, 2021, the entire contents of which are incorporated herein by reference.

### Technical Field

The present invention relates to a method for preparing a key intermediate for synthesizing a sphingosine-1-phosphate receptor agonist.

### BACKGROUND ART

Sphingosine-1-phosphate (S1P) is produced through an intracellular ceramide pathway, and the ceramide starting material of such a synthetic pathway is produced by two production pathways, that is, a de novo biosynthesis pathway and the degradation of sphingomyelin that is a cell membrane constituent in cells. A S1P level in each tissue is controlled by two biosynthesis sphingosine kinases (SphKs) and two biodegradable S1P phosphatases (S1P lyase and lysophospholipid phosphatase), and the S1P that is a material produced by the phosphorylation of sphingosine by sphingosine kinase is known to mediate various cellular reactions such as cell proliferation, cytoskeletal organization and migration, adherence- and tight junction assembly, and morphogenesis. The S1P exists at a high concentration (100-1000 nM) in plasma in a combined form with other plasma proteins including albumin, but exists at a low concentration in tissues.

S1P is combined with an S1P receptor that is a G-protein coupled receptor, and shows various biological functions, and the sub-types of the S1P receptor, known up to now are five of S1P1-S1P5, and these are named endothelial differentiation gene (EDG) receptors 1, 5, 3, 6 and 8. Such S1P receptors are known to engage in various biological functions such as leukocyte recirculation, neural cell proliferation, morphological changes, migration, endothelial function, vasoregulation and cardiovascular development.

In recent years, many studies have found that the S1P signaling process via these receptors plays an important role in a series of responses related to multiple sclerosis, including inflammation response and the repair process, and actually, a non-selective S1P1 agonist has been recently approved as a therapeutic agent for multiple sclerosis. The S1P receptors are extensively expressed in many cells related to the induction of multiple sclerosis. Particularly, the S1P1 receptor plays a very important role in the immune system. The S1P1 receptor is mainly expressed on the surface of lymphocytes such as T cells and B cells and responds to S1P, resulting in involvement in recirculation of lymphocytes. In normal conditions, the S1P concentration is higher in body fluids than in lymphoid tissues, and therefore lymphocytes leave lymphoid tissues by the difference of S1P concentration to circulate along efferent lymph. However, if the S1P1 receptor in lymphocytes is down-regulated by an S1P1 agonist, the egress of lymphocytes from lymphoid tissues does not occur, resulting in reduced infiltration of autoaggressive lymphocytes, which cause inflammation and tissue damage in the central nervous system (CNS). As a result, a therapeutic effect on multiple sclerosis is obtained. Fingolimod, a non-selective S1P1 agonist, has been approved as an oral medication for treating multiple sclerosis. When it binds to the S1P1 receptor and is activated, the receptor becomes degraded or internalized from the surface of lymphocytes. Thus, fingolimod acts as a functional S1P1 antagonist paradoxically.

In relation to the S1P receptor agonist, Korean Laid-open Patent Publication No. 10-2014-0104376 discloses a novel compound of Formula 1 below as an effective S1P receptor agonist.

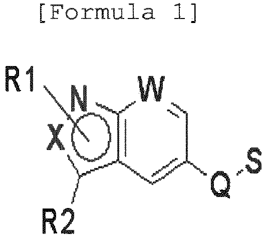

In Formula 1,
X is C or N,
R1 is H or substitutable alkyl,
R2 is H, substitutable alkyl, halogen, CN, CF₃ or COCF₃,
W is C, N, C-alkoxy, C-halogen or C-CN,
Q is CH₂O or 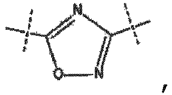 and
S is selected from the following residual groups:

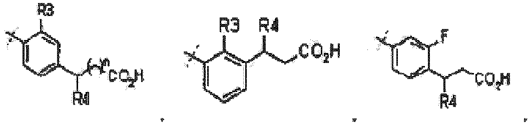

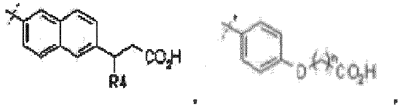

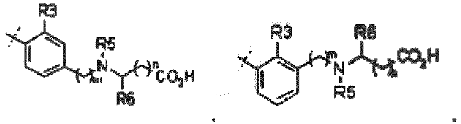

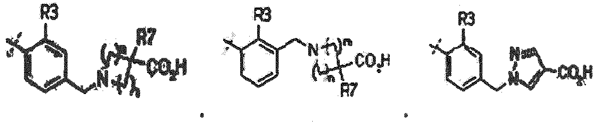

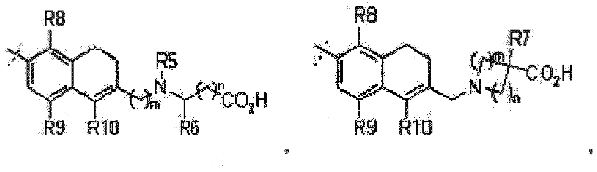

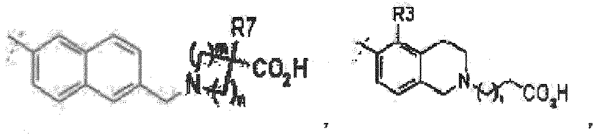

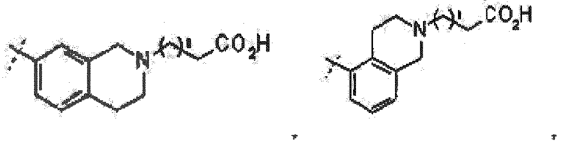

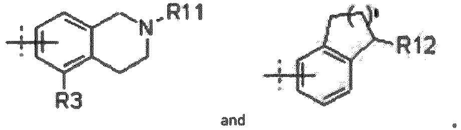

In the above structures,
m, and n are 0, 1, 2 or 3,
R3 to R10 are each H, alkyl, halogen, halogeno-alkyl or alkoxy alkyl,
R11 is H, 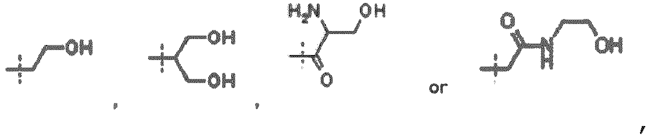
R12 is OH, NH2,

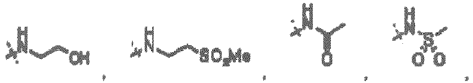

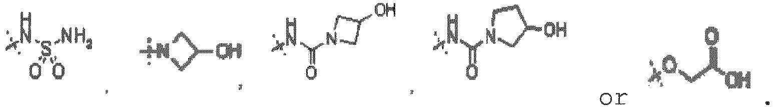

In a particular embodiment of the document, the preparation of 1-[1-chloro-6-(3-chloro-1-isopropyl-1H-indazol-5-ylmethoxy)-3,4-dihydro-naphthalen-2-ylmethyl]piperidin-4-carboxylic acid by Reaction 1 below is disclosed (in Reaction 1, "SG35" refers to "1-chloro-6-hydroxy-3,4-dihydro-naphthalen-2-carbaldehyde").

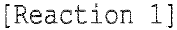

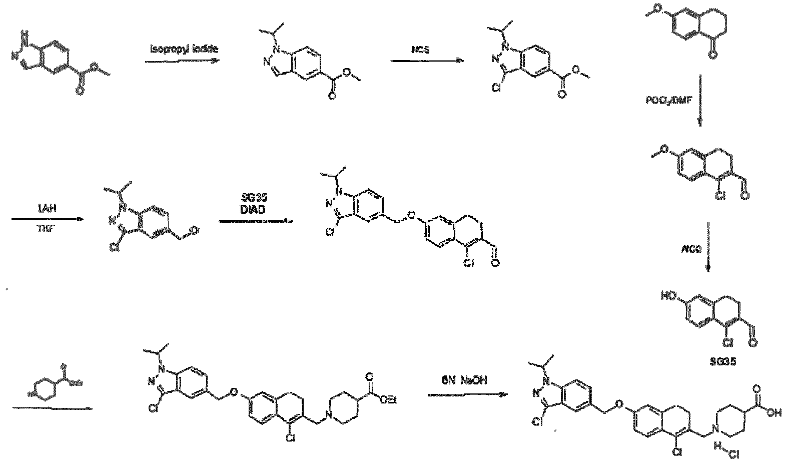

In Reaction 1, a step for preparing 1-chloro-6-(3-chloro-1-isopropyl-1H-indazol-5-ylmethoxy)-3,4-dihydro-naphthalen-2-carbaldehyde will be described in detail.

### (1-1) Synthesis of (3-chloro-1-isopropyl-1H-indazol-5-yl)-methanol

1H-indazol-5-carboxylic acid methyl ester was dissolved in dimethylformamide, and isopropyl iodide and sodium hydride were slowly added dropwisely at 0°C, followed by stirring at 50°C for 8 hours. 1 N hydrochloric acid solution was added thereto, and extraction with ethyl acetate was performed. The resultant was washed with brine, dried over anhydrous magnesium sulfate, and filtered. The filtrate was distilled under a reduced pressure. Through the separation by column chromatography, 1-isopropyl-1H-indazol-5-carboxylic acid methyl ester was obtained.

The 1-isopropyl-1H-indazol-5-carboxylic acid methyl ester thus obtained was dissolved in dimethylformamide, and N-chlorosuccinimide (NCS) was added thereto dropwisely, followed by stirring at room temperature for 18 hours. Water was added thereto, and extraction with ethyl acetate was performed. The resultant was washed with brine, dried over anhydrous magnesium sulfate, and filtered. The filtrate was distilled under a reduced pressure. The residue was separated by column chromatography to obtain 3-chloro-1-isopropyl-1H-indazol-5-carboxylic acid methyl ester.

The 3-chloro-1-isopropyl-1H-indazol-5-carboxylic acid methyl ester thus obtained was dissolved in tetrahydrofuran, and lithium aluminum borohydride was added thereto dropwisely. After stirring at room temperature for 1 hour, water and 6 N sodium hydroxide aqueous solution, and water were added one by one. Celite was added thereto dropwisely, and filtering was performed. The filtrate was distilled under a reduced pressure. The residue was separated by column chromatography to obtain (3-chloro-1-isopropyl-1H-indazol-5-yl)-methanol.

### (1-2) Synthesis of 1-chloro-6-hydroxy-3,4-dihydro-naphthalen-2-carbaldehyde

First, to a solution of 6-methoxy-3,4-dihydronaphthalen-1(2H)-one dissolved in toluene, N,N-dimethylformamide (DMF) and phosphorous oxychloride (POCl₃) were added dropwisely at 0°C, followed by stirring at 70°C for 6 hours. The reaction mixture was poured into ice, and then, extracted with ethyl acetate. An organic layer was washed with brine, dried and concentrated, and the residue thus obtained was purified by silica gel column chromatography (hexane: ethyl acetate = 20:1 to 10:1) to obtain 1-chloro-6-methoxy-3,4-dihydro-2-naphthalencarbaldehyde.

Then, aluminum chloride (AlCl₃) was added at 0°C to a solution of 1-chloro-6-methoxy-3,4-dihydro-2-naphthalene carbaldehyde dissolved in dichloromethane, followed by stirring at 50°C for 6 hours. The reaction mixture was poured into ice, and extraction with ethyl acetate was performed. An organic layer was dried and concentrated, and then the residue thus obtained was purified by silica gel column chromatography (hexane: tetrahydrofuran = 5:1 to 3:1) to obtain 1-chloro-6-hydroxy-3,4-dihydro-2-naphthalene carbaldehyde.

### (1-3) Synthesis of 1-chloro-6-(3-chloro-1-isopropyl-1H-indazol-5-ylmethoxy)-3,4-dihydro-naphthalen-2-carbaldehyde

(3-Chloro-1-isopropyl-1H-indazol-5-yl)-methanol and 1-chloro-6-hydroxy-3,4-dihydro-2-naphthalene carbaldehyde, obtained above, were dissolved in toluene, and then tributyl phosphine (PBu₃) and 1,1'-(azodicarbonyl)dipiperidine (ADD) were added thereto dropwisely. After stirring at room temperature for 18 hours, an excess amount of hexane was added. After filtration and distillation under a reduced pressure, the residue was purified by column chromatography to obtain 1-chloro-6-(3-chloro-1-isopropyl-1H-indazol-5-ylmethoxy)-3,4-dihydro-naphthalen-2-carbaldehyde.

However, the above reactions may have problems in producing clinical APIs as follows.

First, in the process of synthesizing 3-chloro-1-isopropyl-1H-indazol-5-carboxylic acid methyl ester, there may be problems according to the production ratio of N2 isomers. Lithium aluminum hydride (LAH) used for the synthesis of (3-chloro-1-isopropyl-1H-indazol-5-yl)-methanol has disadvantages in that it has very limited stability for use in large scale synthesis and is easily decomposed by water.

In addition, during the Vilsmeier-Haack reaction to obtain 1-chloro-6-methoxy-3,4-dihydro-2-naphthalene carbaldehyde, the exothermic problems may arise as the reaction occurs at a high temperature of 70°C. In addition, in the reaction to obtain 1-chloro-6-hydroxy-3,4-dihydro-2-naphthalene carbaldehyde, there may be problems of reactor contamination due to the use of AlCl₃ or stability problems due to the use of hazardous reagents. If AlCl₃ is used, there are stability problems due to the occurrence of batch fail caused by reaction stop or side reaction progress, and the total yield is 70%, so there is a need to improve the yield.

In addition, 1,1'-(azodicarbonyl)dipiperidine (ADD) which is used for the coupling of (3-chloro-1-isopropyl-1H-indazol-5-yl)-methanol with 1-chloro-6-hydroxy-3,4-dihydro-naphthalen-2-carbaldehyde, is not preferable in terms of low yield problems and cost.

In addition, there are problems in that API reprocessing is required to be performed due to the impurities of the N2 isomers. The synthesis method is a linear process, and there were problems in that the yield is deteriorated whenever each process was performed. In addition, the synthesis method had problems in that it was not easy to identify and solve the causes if problems occurred in the intermediate process. For example, if impurities were not removed during recrystallization, the first step of the process must be started anew.

Accordingly, the inventors of the present invention has invented a new synthesis method as shown in Reaction 2 below for the mass production of an intermediate compound such as 1-chloro-6-(3-chloro-1-isopropyl-1H-indazol-5-ylmethoxy)-3,4-dihydro-naphthalen-2-carbaldehyde, with high yield through a simpler process.

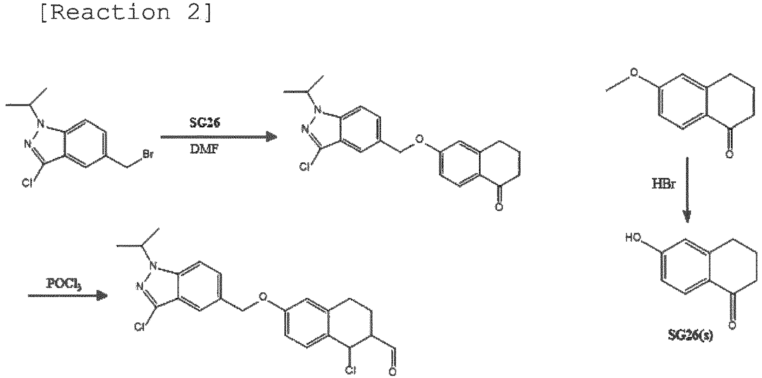

In Reaction 2 above, a step for preparing 1-chloro-6-(3-chloro-1-isopropyl-1H-indazol-6-ylmethoxy)-3,4-dihydro-naphthalen-2-carbaldehyde is described in more detail as follows (in Reaction 2, "SG26" refers to "6-hydroxy-3,4-dihydro-2H-naphthalen-1-one").

### (2-1) Synthesis of 6-hydroxy-3,4-dihydro-2H-naphthalen-1-one

HBr and 6-methoxy-3,4-dihydro-2H-naphthalen-1-one, dissolved in water, were injected into a reactor, followed by refluxed at an external temperature of 120°C for 52 hours. IPC was performed using HPLC and the reaction was completed (3% > 6-methoxy-3,4-dihydro-2H-naphthalen-1-one). The internal temperature was reduced to 10°C, and the resultant solid was filtered. After washing with water and drying with nitrogen, 6-hydroxy-3,4-dihydro-2H-naphthalen-1-one (SG26) was obtained.

### (2-2) Synthesis of 6-(3-chloro-1-isopropyl-1H-indazol-5-ylmethoxy)-3,4-dihydro-2H-naphthalen-1-one

5-Bromomethyl-3-chloro-1-isopropyl-1H-indazole, 6-hydroxy-3,4-dihydro-2H-naphthalen-1-one, K₂CO₃, and DMF were injected into a reactor and reacted at the internal temperature of 25°C for 3 hours. IPC was performed using HPLC, and 5% of 6-hydroxy-3,4-dihydro-2H-naphthalen-1-one remained. So, 5-bromomethyl-3-chloro-1-isopropyl-1H-indazole was additionally injected to complete the reaction (1% > 6-hydroxy-3,4-dihydro-2H-naphthalen-1-one). Water was added to the reactor, the internal temperature was reduced to 0°C, and then the solid thus produced was filtered. The filtered solid was washed sequentially with water and MTBE, respectively, and then dried with nitrogen to obtain 6-(3-chloro-1-isopropyl-1H-indazol-5-ylmethoxy)-3,4-dihydro-2H-naphthalen-1-one.

### (2-3) Synthesis of 1-chloro-6-(3-chloro-1-isopropyl-1H-indazol-6-ylmethoxy)-3,4-dihydro-naphthalen-2-carbaldehyde

Phosphoryl chloride (POCl₃) was injected into a reactor, and the internal temperature was reduced to 0°C. DMF was slowly added dropwisely, stirring at the internal temperature of 50°C was performed for 2 hours, and 6-(3-chloro-1-isopropyl-1H-indazol-5-ylmethoxy)-3,4-dihydro-2H-naphthalen-1-one was injected and reacted at the internal temperature of 50°C for 3 hours. Excessive HCl gas might be generated during the reaction, and a vent line was installed so that excessive HCl gas could be neutralized by a NaOH trap. IPC was performed using HPLC and the reaction was completed. The internal temperature was reduced to 0°C, and then cold water, hexane (Hex), and MTBE were injected to another reactor. The above reaction mixture was slowly injected thereto dropwisely for 90 minutes to form crystals. The solid thus obtained was filtered, washed sequentially with water and a mixed solvent of MTBE/HEX, respectively, and dried to obtain 1-chloro-6-(3-chloro-1-isopropyl-1H-indazol-6-ylmethoxy)-3,4-dihydro-naphthalen-2-carbaldehyde.

There were attempts to improve the problems of the conventional synthesis method through the above-described synthesis methods, but there were problems of arising low yield due to the decomposition of a benzyl group during Vilsmeier-Haack Reaction and of producing various by-products.

Accordingly, the development of a new synthesis method for converting the conventional linear process into effective convergent synthesis is still required.

### DISCLOSURE OF THE INVENTION

### TECHNICAL PROBLEM

Therefore, an object of the present invention is to provide a suitable method for producing a compound of Formula 4 that is a key intermediate in the novel synthesis method of an excellent sphingosine-1-phosphate receptor agonist with high yield.

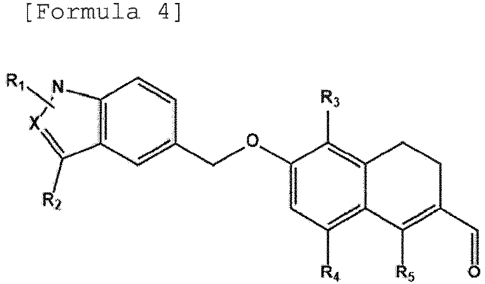

In Formula 4,
R1 is hydrogen, or substituted or unsubstituted alkyl,
R2 is hydrogen, substituted or unsubstituted alkyl, halogen, CN, CF₃, or COCF₃,
R3 and R4 are each hydrogen, substituted or unsubstituted alkyl, or halogen,
R5 is hydrogen, substituted or unsubstituted alkyl, or halogen,
X is C or N, and
L is a leaving group.

### TECHNICAL SOLUTION

In order to accomplish the above-described objects,
in an aspect according to the present invention provides a method for preparing an intermediate compound of Formula 4, comprising a step of preparing the intermediate compound of Formula 4 by the coupling reaction of a compound of Formula 2 with a compound of Formula 3.

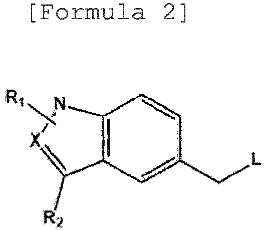

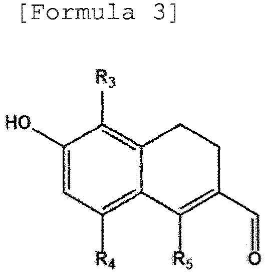

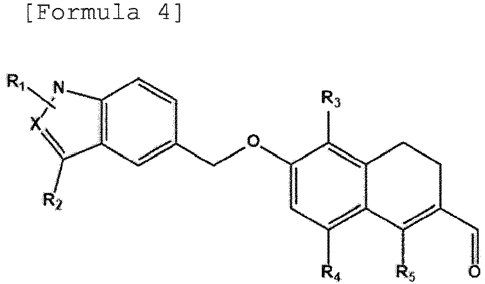

In Formulae 2 to 4,
R1 is hydrogen, or substituted or unsubstituted alkyl,
R2 is hydrogen, substituted or unsubstituted alkyl, halogen, CN, CF₃, or COCF₃,
R3 and R4 are each hydrogen, substituted or unsubstituted alkyl, or halogen,
R5 is hydrogen, substituted or unsubstituted alkyl, or halogen,
X is C or N, and
L is a leaving group.

In the alkyl where "alkyl" is substituted, the substituent may be one or more, and the substituents may be each independently selected from the group consisting of halogen, cyano, hydroxyl, alkyloxy, oxo, unsubstituted sulfonyl and alkyl-substituted sulfonyl.

According to an embodiment of the present invention, R1 in the above formulae may be hydrogen, or C₁-C₆ substituted or unsubstituted alkyl, R2 may be hydrogen, C₁-C₆ substituted or unsubstituted alkyl, halogen, CN, CF₃, or COCF₃. R3 and R4 may be each hydrogen, or C₁-C₆ substituted or unsubstituted alkyl. R5 may be F, Cl, Br, or I.

According to another embodiment of the present invention, R1 may be C₁-C₄ substituted or unsubstituted alkyl, R2 may be halogen (F, Cl, Br or I). R3 and R4 may be each hydrogen. R5 may be Cl.

According to an embodiment of the present invention, the leaving group (L) is a reacting group providing the compound of Formula 2 with a substitution position during the substitution reaction of the compound of Formula 2 and an alcohol-based compound such as Formula 3, and may be selected from chlorine (Cl), bromine (Br), iodine (I), methanesulfonate (Oms), p-toluenesulfonate (OTs) and trifluoromethanesulfonate (OTf), without limitation.

According to another embodiment of the present invention, L may be Br.

In the present invention, the compound of Formula 4 is prepared by the coupling reaction of the compound of Formula 2 with the compound of Formula 3.

In an embodiment according to the present invention, the coupling reaction of the compound of Formula 2 with the compound of Formula 3 may be easily performed by using K₂CO₃ in a dimethylformamide (DMF) solvent.

In another embodiment according to the present invention, a step for crystallization may be included after the coupling reaction of the compound of Formula 2 with the compound of Formula 3 to obtain the compound of Formula 4 with high purity.

In an embodiment according to the present invention, there may be problems of not performing the crystallization according to the solvent used during crystallizing the compound of Formula 4, and the solvent for crystallizing the compound of Formula 4 may preferably include an alcohol-based solvent. For example, during crystallizing the compound of Formula 4, crystallization may not be performed if using water or methyl tert-butyl ether (MTBE) solely.

The "alcohol-based solvent" for crystallizing the compound of Formula 4 may be, for example, one or more solvents selected from methanol, ethanol, isopropyl alcohol and butanol, without limitation.

In an embodiment according to the present invention, the solvent for the crystallization may be a mixture solvent of an alcohol-based solvent and water. By using the mixture solvent of the alcohol-based solvent and water as the solvent for crystallization, effects of crystallizing the compound of Formula 4 and obtaining the compound of Formula 4 with high yield may be achieved.

In another embodiment according to the present invention, the mixture solvent for crystallization may use the alcohol-based solvent and water in a volume ratio of 5:1 to 1:5, 4:1 to 1:4, 3:1 to 1:3, 2:1 to 3:1, 1:1 to 3:1, 1:1 to 2.5:1, or 1:2.5 in view of the yield of Formula 4.

In an embodiment according to the present invention, the solvent for crystallization may be a mixture solvent of ethanol and water. Particularly, ethanol and water may be used in a volume ratio of ETOH: H₂O of 5:1 to 1:5, 4:1 to 1:4, 3:1 to 1:3, 2:1 to 3:1, 1:1 to 3:1, 1:1 to 2.5:1, or 1:2.5.

According to another aspect of the present invention, the compound of Formula 3 may be prepared from a compound of Formula 5.

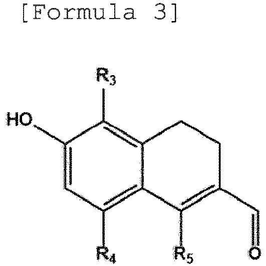

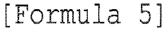

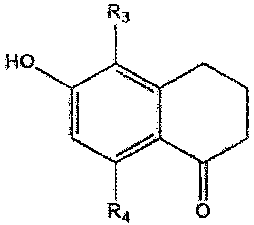

Particularly, the compound of Formula 3 may be obtained by introducing an aldehyde into the compound of Formula 5.

In an embodiment according into the present invention, the reaction for introducing an aldehyde into the compound of Formula 5 may be performed using the reagents for the Vilsmeier-Haack reaction.

In another embodiment according to the present invention, the reaction for introducing an aldehyde into the compound of Formula 5 may include reacting the compound of Formula 5 with phosphoryl chloride (POCl₃) and dimethylformamide (DMF) to prepare the compound of Formula 3.

In an embodiment according to the present invention, the reaction for introducing an aldehyde into the compound of Formula 5 may be performed at a temperature of 30°C or less to show stabilizing effects of exothermic problems due to the Vilsmeier-Haack reaction.

In another embodiment according to the present invention, the reaction for introducing an aldehyde into the compound of Formula 5 may be performed at a temperature of 0°C to 25°C.

In another embodiment according to the present invention, the reaction for introducing an aldehyde into the compound of Formula 5 may be performed by the steps below.
1) a step of blocking the alcohol group of the compound of Formula 5 to prepare a compound of Formula 6;
2) a step of introducing an aldehyde into the compound of Formula 6 to prepare a compound of Formula 7; and
3) a step of restoring the alcohol group from the compound of Formula 7 to prepare the compound of Formula 3.

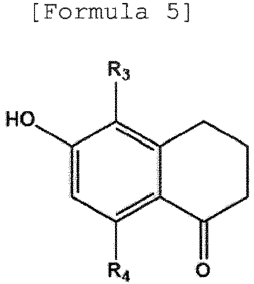

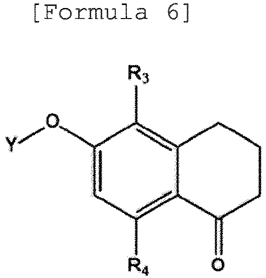

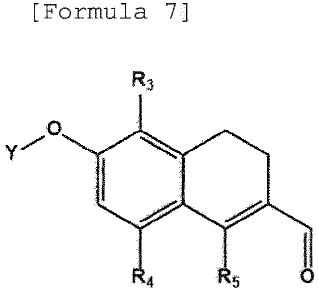

In Formula 7, Y is an alcohol-protecting group.

In the present invention, an aldehyde may be introduced without blocking the alcohol group of the compound of Formula 5, and the compound of Formula 3 may be prepared by simplifying a process, but in view of the yield of the compound of Formula 3, the aldehyde introduction reaction may be performed after blocking the alcohol group of the compound of Formula 5 with an alcohol-protecting group.

The blocking of the alcohol group in the compound of Formula 5 may be performed by a known alcohol blocking method, and the method is not specifically limited.

Accordingly, an alcohol-protecting group (Y) to be introduced into the compound of Formula 5 is not specifically limited as long as it is a known alcohol-protecting group, and may include, for example, an acetyl group (-Ac), a trimethylsilyl group (-TMS), a tert-butyldimethylsilyl group (-TBDMS), or the like, without limitation.

The step of introducing an aldehyde into the compound of Formula 6 of which alcohol is blocked to prepare the compound of Formula 7, may be performed by the same method as the step of introducing an aldehyde into the compound of Formula 5 of which alcohol group is not blocked.

Then, after preparing the compound of Formula 7 by introducing an aldehyde into the compound of Formula 6, the alcohol group may be restored using a suitable alcohol de-blocking agent according to the alcohol-protecting group to prepare the compound of Formula 3.

According to an embodiment of the present invention, the alcohol de-blocking agent may use a known base substance, for example, K₂CO₃, NaHCO₃, and NaOH, without limitation.

In the present invention, the reaction solvent may be suitably selected according to the type of the de-blocking agent used during the alcohol restoration reaction.

According to an embodiment of the present invention, if the base substance such as K₂CO₃, NaHCO₃, and NaOH is used as the alcohol de-blocking agent, the reaction solvent may use an alcohol-based solvent, for example, methanol, ethanol or a mixture solvent thereof.

According to another embodiment of the present invention, if one or more among K₂CO₃ and NaHCO₃ are used as the alcohol de-blocking agents, and if the reaction solvent is methanol, excellent effects may be shown in view of the reaction time and yield of the alcohol restoration reaction.

According to an embodiment of the present invention, the compound of Formula 3 may be prepared by blocking an alcohol group in the compound of Formula 5 using acetyl chloride (AcCl), introducing an aldehyde group using POCl₃ and DMF, and then using K₂CO₃ in the presence of an alcohol-based solvent.

According to another embodiment of the present invention, the compound of Formula 3 may be prepared by blocking an alcohol group in the compound of Formula 5 using acetyl chloride (AcCl), introducing an aldehyde group using POCl₃ and DMF, and then using K₂CO₃ in the presence of a methanol solvent.

In the present invention, through crystallizing the compound of Formula 3 thus prepared, the compound of Formula 3 with high purity may be obtained.

According to an embodiment of the present invention, since the compound of Formula 3 is prepared through a series of steps without a purification process between the steps, the purity may be improved through crystallization.

In the present invention, the crystallization of the compound of Formula 3 may be performed under acidic conditions. The acidic conditions may be, for example, conditions of pH 4.0 or less. Particularly, crystallization may be performed by injecting an acid compound so that the pH of a solution containing the compound of Formula 3 becomes 3.0 to 4.0.

The acid compound used for forming the acidic conditions may use a known acid compound within a range not damaging the structure and physical properties of the compound of Formula 3, and the type of the acid compound is not limited.

According to an embodiment of the present invention, the crystallization may be performed by injecting hydrochloric acid (HCl) to the reaction product containing the compound of Formula 3.

According to another embodiment of the present invention, the crystallization may be performed by injecting water (H₂O) and hydrochloric acid (HCl) simultaneously or sequentially to the reaction product containing the compound of Formula 3.

According to another embodiment of the present invention, the crystallization may be performed by injecting water and then hydrochloric acid to the reaction product containing the compound of Formula 3.

According to an embodiment of the present invention, the reaction for restoring the alcohol group may be finished, and then, the crystallization of the compound of Formula 3 may be performed by distilling off the reaction solvent, for example, methanol.

According to another embodiment of the present invention, the crystallization of the compound of Formula 3 may be performed without distilling off the reaction solvent, for example, methanol, after finishing the restoration reaction of the alcohol group. By performing the crystallization without the reaction solvent in the restoration step of the alcohol group, the loss of the compound of Formula 3 may be reduced, and improving effects of yield may be shown.

According to an embodiment of the present invention, the reaction temperature during crystallizing the compound of Formula 3 may be 10°C or less.

According to an embodiment of the present invention, the reaction temperature during crystallizing the compound of Formula 3 may be 10°C or less, and the dropwise addition of hydrochloric acid may be performed for less than 2 hours, for example, within 1 hour, 30 minutes, 20 minutes, or 10 minutes.

In another embodiment of the present invention, the compound of Formula 5 may be prepared by the dealkylation of a compound of Formula 8.

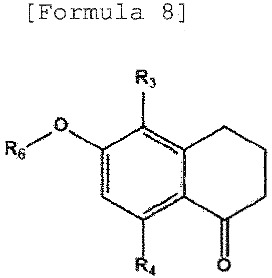

In Formula 8, R3 and R4 are the same as defined above, and R6 is substituted or unsubstituted alkyl.

In an embodiment of the present invention, R6 may be C₁-C₄ unsubstituted alkyl.

In another embodiment of the present invention, R6 may be a methyl group.

In an embodiment of the present invention, the compound of Formula 3 may be prepared by introducing an aldehyde into the compound of Formula 8 according to the same method as the method for introducing an aldehyde into the compound of Formula 5 or the compound of Formula 6, but by preparing the compound of Formula 3 by performing the dealkylation from the compound of Formula 8 to prepare the compound of Formula 5, and introducing an aldehyde into the compound of Formula 5 or Formula 6, the problems of the decomposition of the compound of Formula 8 may be improved, and an intermediate compound may be prepared with high yield even in mass production.

The dealkylation of the compound of Formula 8 may be performed by a known method for preparing the compound of Formula 5 through substituting the alkyl group of R6 with an alcohol group, and the method and the type of reagents used are not specifically limited.

In an embodiment of the present invention, the dealkylation reaction of the compound of Formula 8 may be performed by using, for example, one selected from hydrogen bromide (HBr), aluminum chloride (AlCl₃), and iron(III) chloride (FeCl₃), without limitation.

The compound prepared according to the present invention may be used as the key intermediate for synthesizing a sphingosine-1-phosphate receptor agonist. The compound prepared according to the present invention may be used as the key intermediate in the known synthesis method of a sphingosine-1-phosphate receptor agonist, and may be used as the key intermediate in a novel synthesis method to be developed after the present application. The use of the present invention is not limited to a specific synthesis method for a sphingosine-1-phosphate receptor agonist.

In addition, the compound prepared according to the present invention may be used in other uses in addition to the synthesis of the sphingosine-1-phosphate receptor agonist, and the use of the present invention is not limited to the synthesis of the sphingosine-1-phosphate receptor agonist.

### ADVANTAGEOUS EFFECTS

By using the preparation method of the present invention, effects of the mass production of the compound of Formula 4 with high yield may be achieved.

### BEST MODE FOR CARRYING OUT THE INVENTION

Hereinafter, embodiments will be described below in more detail with reference to examples to assist the understanding of the present invention. The embodiments according to the present inventive may, however, be embodied in different forms and should not be construed as limited to the embodiments set forth herein. These embodiments of the present invention are provided so as to explain the present invention more completely to those having average knowledge in the field to which the present invention belongs.

### [Example 1. Synthesis of 5-bromomethyl-3-chloro-1-isopropyl-1H-indazole]

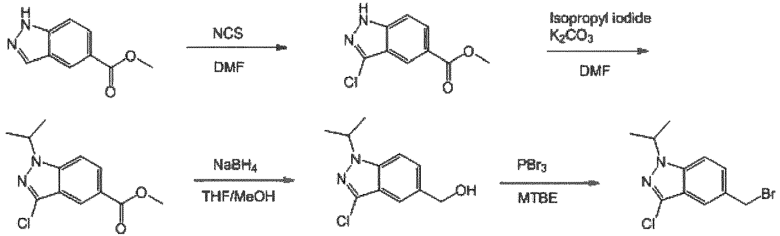

### Preparation Example 1-1. Synthesis of 3-chloro-1-isopropyl-1H-indazol-5-carboxylic acid methyl ester

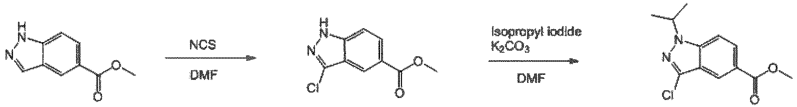

1H-Indazol-5-carboxylic acid methyl ester) (6.2 kg, 35.19 mol), N-chlorosuccinimide (NCS, 5.64 kg, 42.2 mol), and dimethylformamide (DMF, 31.0 ml, 5 fold) were injected into a reactor, and the internal temperature of the reaction mixture was raised to 75°C, followed by reacting for 1.5 hours.

Reaction ion-pair chromatography (IPC) was performed by HPLC, and after finishing the reaction (1H-indazol-5-carboxylic acid methyl ester: N/D), the external temperature was set to 0°C for cooling for 60 minutes. While maintaining the internal temperature of the reactor to 50°C, K₂CO₃ (10.7 kg, 77.4 mol) and 2-iodopropane (8.98 kg, 52.8 mol) were added, and alkylation was performed at 60°C for 120 minutes.

As a result of performing the reaction IPC by HPLC, 19.3% of methyl 3-chloro-1H-indazol-5-carboxylate was remained, and K₂CO₃ (2.14 kg, 15.5 mol) and 2-iodopropane (1.80 kg, 10.6 mol) were additionally injected twice. 2.4% of methyl 3-chloro-1H-indazol-5-carboxylate was remained, and K₂CO₃ (1.08 kg, 7.75 mol) and 2-iodopropane (0.9 kg, 5.3 mol) were additionally injected, and the reaction was terminated (1% > methyl 3-chloro-1H-indazol-5-carboxylate).

The reaction mixture was cooled to 30°C, and water (43.4 L) and EtOAc (43.4 L) were injected, followed by stirring for 30 minutes. Layers were separated, and an aqueous layer was removed. Water (31.0 L) was injected, and an organic layer was additionally washed. EtOAc was removed by distillation under a reduced pressure, EtOH (24.8 L) was injected, the temperature was raised to 40°C, and the resultant was heated until a clear solution was obtained. The reactor was cooled to maintain the internal temperature to 20°C, and then, water (24.8 L) was slowly added dropwisely to produce crystals. The solid thus produced was aged for 30 minutes, then filtered, washed with water (31.0 L) twice, and dried under nitrogen to obtain the title compound (6.56 kg, Net yield 64.9%).

¹H NMR (400MHz, CDCl₃): 1.58 (d, 6H), 3.96 (s, 3H), 4.81 (m, 1H), 7.42 (d, 1H), 8.06 (dd, 1H), 8.44 (s, 1H).

### Preparation Example 1-2. Synthesis of (3-chloro-1-isopropyl-1H-indazol-5-yl)-methanol

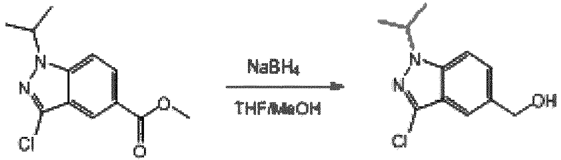

To a reactor, THE (34.2 L, 6 fold) and 3-chloro-1-isopropyl-1H-indazol-5-carboxylic acid methyl ester (Example 1-1, 5.7 kg, 22.6 mol) were injected, and the internal temperature was raised to 60°C. To the reaction mixture, NaBH₄ (1.68 kg, 44.4 mol) was added, and MeOH (5.7 L, 1 fold) was slowly added dropwisely over 80 minutes, followed by reacting for 30 minutes. With an interval of 90 minutes, NaBH₄ (0.44 kg, 11.6 mol) was added, MeOH (1.69 L, 0.3 fold) was injected twice, the reaction was performed for 30 minutes, and IPC was performed using HPLC.

14.3% of 3-chloro-1-isopropyl-1H-indazol-5-carboxylic acid methyl ester was remained, and only NaBH₄ (0.22 kg, 5.8 mol) was additionally injected, followed by reacting for 120 minutes. 2.5% of 3-chloro-1-isopropyl-1H-indazol-5-carboxylic acid methyl ester was remained, and the reaction was terminated, and the resultant was cooled to set the internal temperature to 10°C.

In order to remove a B-complex (produced by NaBH₄ and a complex in which boron is conjugated with the alcohol of (3-chloro-1-isopropyl-1H-indazol-5-yl)-methanol)), and remaining NaBH₄, 3N HCl (39.3 kg) was slowly injected over 60 minutes, the pH of the reaction solution was maintained to 3.0, and the solvent was removed by distillation under a reduced pressure.

DCM (28.5 L) and water (57.0 L) were injected to the residue, layer separation was performed, an aqueous layer was removed, additional washing was performed with water (42.8 L), and distillation under a reduced pressure was performed to obtain the title compound (4.32 kg, Net yield 85%).

¹H NMR (400MHz, CDCl₃): 1.5-1.7 (m, 6H), 1.82 (m, 1H), 3.72 (m, 1H), 4.70-5.10 (m, 2H), 7.30-7.50 (m, 2H), 7.62 (s, 1H) .

### Example 1. Synthesis of 5-bromomethyl-3-chloro-1-isopropyl-1H-indazole

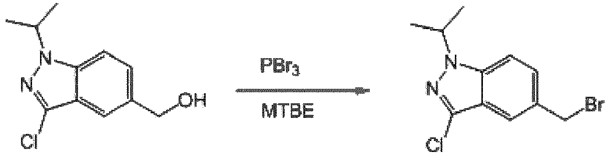

To a reactor, MTBE (43.3 L, 8 fold), and (3-chloro-1-isopropyl-1H-indazol-5-yl)-methanol (Example 1-2, 4.32 kg, 19.3 mol) were injected, and the internal temperature was cooled to 0°C. To the reaction mixture, PBr₃ (3.64 kg, 13.5 mol) was slowly injected over 90 minutes, and the reaction was performed for 180 minutes.

IPC was performed using HPLC, and the reaction was completed (Example 1-2: N/D). 1.5 N of NaOH (34.7 L) was slowly injected over 60 minutes, followed by stirring for 30 minutes to terminate the reaction. To the reaction mixture, water (21.7 L) was injected, stirring and layer separation were performed for 10 minutes, an aqueous layer was removed, additional washing with water (17.3 L) was performed. Then, an organic layer was distilled under a reduced pressure to synthesize the title compound (4.97 g, Net yield 90.0%).

¹H NMR (400MHz, CDCl₃): 1.53 (d, 6H), 4.7 (s, 2H), 4.88 (m, 1H), 7.51-7.6 (m, 2H), 7.68 (s, 1H).

### [Example 2. Synthesis of 1-chloro-6-hydroxy-3,4-dihydronaphthalen-2-carbaldehyde]

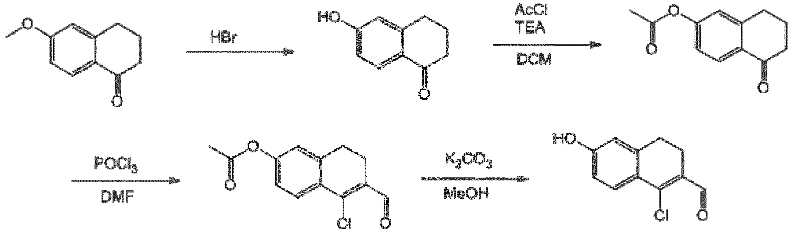

### Preparation Example 2-1. Synthesis of 6-hydroxy-3,4-dihydro-2H-naphthalen-1-one

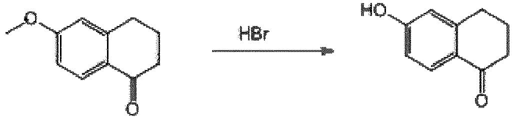

To a reactor, HBr in water (HBr in H₂O) (47.2 L, 10 fold), and 6-methoxy-3,4-dihydro-2H-naphthalen-1-one (4.72 kg, 26.8 mol) were injected and refluxed at an external temperature of 100°C for 16.5 hours.

IPC was performed using HPLC, the reaction was completed (6-methoxy-3,4-dihydro-2H-napthalen-1-one: 0.72%). The internal temperature was cooled to 10°C, and the solid thus produced was filtered. By washing with water (23.6 L) twice, and drying under nitrogen, the title compound (4.22 kg, Net yield 92.9%) was synthesized.

¹H NMR (400MHz, DMSO): 1.94-1.98 (m, 2H), 2.48 (m, 2H), 2.81 (t, 2H), 6.62 (d, 1H), 6.68-6.70 (m, 1H), 7.72 (d, 1H).

### Preparation Example 2-2. Synthesis of 5-oxo-5,6,7,8-tetrahydronaphthalen-2-yl acetate

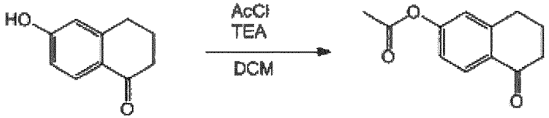

To a reactor, 6-hydroxy-3,4-dihydro-2H-naphthalen-1-one (4.22 kg, 26.0 mol) and DCM (20.2 L, 5 fold) were injected, and the internal temperature was cooled to 0°C. AcCl (2.14 kg, 27.3 mol) was added dropwisely at 10°C or less, and after finishing the injection, TEA (3.02 kg, 29.8 mol) was added dropwisely at 10°C or less, and the reaction was performed.

The internal temperature was raised to 20°C, and the reaction was performed for 1 hour. Then, IPC was performed by HPLC, and the reaction was completed (6-hydroxy-3,4-dihydro-2H-naphtalen-1-one: 0.6%). Water (20.2 L) was injected to the reaction product, and stirring was performed for 10 minutes for layer separation. An aqueous layer was removed, and additional washing with water (20.2 L) was performed. After distilling under a reduced pressure, the title compound (4.56 kg, Net yield 90.0%) was synthesized.

¹H NMR (400MHz, CDCl₃): 2.10 (m, 2H), 2.30 (s, 3H), 2.60 (t, 2H), 2.95 (t, 2H), 7.00 (s, 1H), 7.05 (d, 1H), 8.05 (d, 1H).

### Preparation Example 2-3. Synthesis of 5-chloro-6-formyl-7,8-dihydronaphthalen-2-yl acetate

To a reactor, POCl₃ (10.26 kg, 66.9 mol) was injected, the internal temperature was cooled to 0°C, and DMF (8.16 kg, 111.6 mol) was slowly added dropwisely at 25°C or less, followed by stirring for 60 minutes. To the reactor, 5-oxo-5,6,7,8-tetrahydronaphthalen-2-yl acetate (4.56 kg, 22.3 mol) diluted in DMF (1.64 kg, 22.4 mol) was slowly added dropwisely at 30°C or less. After finishing the dropwise addition, the reaction was performed for 4 hours.

IPC was performed by HPLC, the reaction was completed (5-oxo-5,6,7,8-tetrahydronaphthalen-2-yl acetate: 0.4%) to terminate the reaction. To another reactor, water (45.6 L, 10 fold) was injected, and the internal temperature was cooled to 0°C. The reaction mixture was diluted in EtOAc (31.0 L, 7 fold), and slowly added dropwisely so that the temperature was not raised over 25°C to decompose remaining POCl₃ to quench the reaction.

Layer separation was performed, and an organic layer was collected in another reactor. An aqueous layer was additionally extracted with EtOAc (13.7 L, 3 fold), the aqueous layer was removed, and the remaining layer was injected to the reactor containing the first organic layer. The organic layer thus collected was washed with water (22.8 L, 5 fold), and distilled under a reduced pressure to synthesize the title compound (4.74 kg, Net yield 85.0%).

¹H NMR (500MHz, CDCl₃): 2.30 (s, 3H), 2.65 (t, 2H), 2.85 (t, 2H), 7.00 (s, 1H), 7.05 (m, 1H), 7.90 (d, 1H), 10.40 (s, 1H).

### Example 2. Synthesis of 1-chloro-6-hydroxy-3,4-dihydronaphthalen-2-carbaldehyde

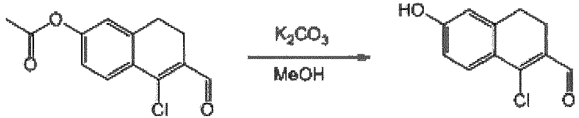

To a reactor, MeOH (22.8 L, 5 fold), and K₂CO₃ (2.52 kg, 18.2 mol) were injected, and the reaction was performed at the internal temperature of 30°C or less for 2 hours. IPC was performed by HPLC, and since 5-chloro-6-formyl-7,8-dihydronaphthalen-2-yl acetate was remained (5-chloro-6-formyl-7,8-dihydronaphthalen-2-yl acetate: 2.7%), the reaction was additionally performed for 1 hour.

After performing IPC by HPLC, the reaction was finished (5-chloro-6-formyl-7,8-dihydronaphthalen-2-yl acetate: 0.75%). The internal temperature was cooled to 5°C, and 3N HCl (18.9 L, 56.7 mol) and water (23.7 L, 5 fold) were added dropwisely to perform crystallization. The crystals thus produced was aged for 60 minutes, filtered, washed with water (23.7 L, 5 fold) and dried under nitrogen to synthesize SG35 (4.98 kg, 4 Step net yield 81.3%).

¹H NMR (400MHz, DMSO): 2.48-2.52 (m, 4H), 6.71 (s, 1H), 6.77 (d, 1H), 7.66 (d, 1H), 10.20 (s, 1H), 10.28 (s, 1H).

### Example 3. Synthesis of 1-chloro-6-(3-chloro-1-isopropyl-1H-indazol-5-ylmethoxy)-3,4-dihydro-naphthalen-2-carbaldehyde

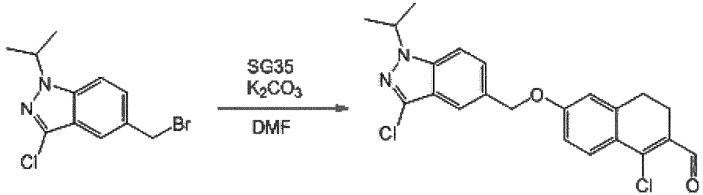

To a reactor, 5-bromomethyl-3-chloro-1-isopropyl-1H-indazole (Example 1, 4.96 kg, 17.2 mol), 1-chloro-6-hydroxy-3,4-dihydronaphthalen-2-carbaldehyde (Example 2, 3.56 kg, 17.1 mol), K₂CO₃ (3.62 kg, 26.2 mol), and DMF (13.1 L, 4 fold) were injected and reacted at the internal temperature of 30°C for 4 hours and 50 minutes.

After performing IPC by HPLC and completing the reaction (1-chloro-6-hydroxy-3,4-dihydronaphthalen-2-carbaldehyde: 0.06%), the internal temperature was cooled to 10°C. To the reaction mixture, a mixture of EtOH (13.1 L, 4 fold), and water (32.6 L, 10 fold) was slowly injected to perform crystallization. After finishing the injection, the internal temperature was cooled to 0°C, aging was performed for 1 hour, and the solid thus produced was filtered. The solid thus filtered were washed with water (16.4 L) and n-Hexane (16.3 L), and dried under nitrogen. After drying, residual moisture was checked using a K/F apparatus (moisture content: 0.20%). Finally, the title compound (6.36 kg, Net yield 89.7%) was synthesized.

¹H NMR (500MHz, CDCl₃): 1.57 (d, 6H), 2.62 (m, 2H), 2.80 (t, 2H), 4.79 (m, 1H), 5.19 (s, 2H), 6.82-6.93 (m, 2H), 7.42-7.50 (m, 2H), 7.71(s, 1H), 7.80 (d, 1H), 10.33 (s, 1H).

### Experimental Example 1. Evaluation on de-blocking agent and solvent during alcohol group restoration reaction (de-acetyl reaction) of 5-chloro-6-formyl-7,8-dihydronaphthalen-2-yl acetate

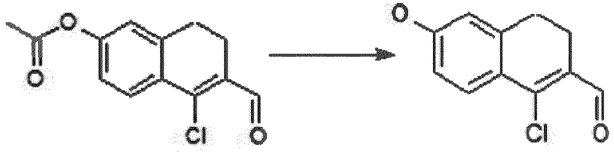

In order to obtain the 1-chloro-6-hydroxy-3,4-dihydronaphthalen-2-carbaldehyde of Example 2 by performing the restoration reaction of an alcohol group with respect to the 5-chloro-6-formyl-7,8-dihydronaphthalen-2-yl acetate obtained in Preparation Example 2-3, without a separate purification process (crude), yield evaluation according to various bases and solvents as de-blocking agents were performed, and the evaluation results are shown in Table 1.

In Table 1, "SG28" represents "5-chloro-6-formyl-7,8-dihydronaphthalen-2-yl acetate", and "SG35" represents "1-chloro-6-hydroxy-3,4-dihydronaphthalen-2-carbaldehyde".

**[Table 1]**

| Entr y | Base (equiv.) | Solven t | Temp (°C) | HPLC PAR (%) | | Note |
|---|---|---|---|---|---|---|
| | | | | SG28 | SG35 | |
| 1 | K2CO3 (3) | Toluen e | 23 | 40.0 | 60.0 | - |
| 2 | K2CO3 (3) | Toluen e | 100 | 7.5 | 92.5 | - |
| 3 | K2CO3 (1) | MeOH | 23 | N/D | 100.0 | Initial SG28 7.5 % |
| 4 | 1N NaOH (1) | MeOH | 23 | N/D | 100.0 | Initial SG28 7.5 % |
| 5 | NaHCO3 (1) | MeOH | 23 | N/D | 100.0 | Initial SG28 7.5 % |
| 6 | K2CO3 (1) | EtOAc | 23 | N/D | 100.0 | Initial SG28 7.5 % |
| 7 | NaHCO3 (2) | MeOH | 50 | N/D | > 99 | Reaction termination at 1 h |
| 8 | K2CO3 (2) | MeOH | 50 | N/D | > 99 | Reaction termination at 1 h |
| 9 | NaOAc (2) | MeOH | 50 | N/D | 98.1 | Reaction termination at 7.5 h |
| 10 | K2CO3 (2) | EtOAc | 50 | N/D | 35.1 | Reaction not terminated even at 7.5 h |
| 11 | 1N NaOH (2) | EtOAc | 50 | N/D | 2.2 | Little conversion even at 7.5 h |

As confirmed in Table 1 above, in case where a K₂CO₃ base and a toluene solvent were used as de-blocking agents, it was confirmed that an alcohol group-blocked substance remained, and even at a raised temperature of 100°C, it was confirmed that the alcohol group-blocked substance still remained. Meanwhile, if a methanol solvent was used, it could be confirmed that the alcohol group could be restored by performing the reaction using a base of K₂CO₃, NaHCO₃, and NaOH, and if an ethyl acetate solvent was used, it could be confirmed that the alcohol group could be restored by performing the reaction using a base of K₂CO₃.

In addition, under a methanol solvent, it was confirmed that the reaction was terminated within 1 hour with both NaHCO₃ and K₂CO₃, and on the contrary, 98% or higher conversion was shown in the case of using NaOAc, but the reaction time was consumed for 7 hours or more. Also, under an ethyl acetate solvent, it was confirmed that the reaction was not terminated even after 7 hours in the case of using K₂CO₃, and the reaction was performed little in the case of using 1 N NaOH.

Through the results, it was confirmed that the use of K₂CO₃ under a methanol solvent was the most effective for the restoration reaction of the alcohol of 5-chloro-6-formyl-7,8-dihydronaphthaln-2-yl acetate. Particularly, in the case of K₂CO₃, it was confirmed that the reaction was completed at room temperature even using 1.1 equivalents.

### Experimental Example 2. Evaluation of crystallization conditions of 1-chloro-6-hydroxy-3,4-dihydronaphthalen-2-carbaldehyde 1

As described above, when preparing 1-chloro-6-hydroxy-3,4-dihydronaphthalen-2-carbaldehyde (Example 2) through Preparation Example 2-1 to Preparation Example 2-3, the preparation could be proceeded without purification and crystallization processes (crude) of each of Preparation Example 2-1 to Preparation Example 2-3, and in order to increase the yield of 1-chloro-6-hydroxy-3,4-dihydronaphthalen-2-carbaldehyde, the crystallization conditions of 1-chloro-6-hydroxy-3,4-dihydronaphthalen-2-carbaldehyde were evaluated.

Particularly, the crystallization of 1-chloro-6-hydroxy-3,4-dihydronaphthalen-2-carbaldehyde in acidic conditions was confirmed, and the crystallization conditions were evaluated through an acidification step using water and 3 N HCl, and the results are shown in Table 2.

**[Table 2]**

| Entry | MeOH distillatio n | Conditions (fold) | Net. Yield (%) |
|---|---|---|---|
| 1 | O | H₂O (5) + 3N HCl (3 equiv.) → EtOAc (1)/n-Hex (3) | 75.0 |
| 2 | O | H₂O (5) + 3N HCl (3 equiv.) → MTBE (3)/n-Hex (3) | 32.1 (gross) |
| 3 | O | H₂O (5) + 3N HCl (3 equiv .) → n-Hex (6) | 74.6 |
| 4 | X | H₂O (5) + 3N HCl (3 equiv.) → n-Hex (10) | 86.6 |
| 5 | X | H₂O (5) + 3N HCl (2 equiv.) | 83.0 |
| 6 | X | H₂O (10) + 3N HCl (2 equiv.) | 78.2 |
| 7 | X | H₂O (5) + 3N HCl (3 equiv.) | 83.9 |

As confirmed in Table 2, if the crystallization was performed after distilling the reaction solvent of methanol (MeOH) in the alcohol restoration reaction, it was confirmed that loss into an aqueous layer occurred, and the crystallization was performed with low yield of 70% level (#1, #2, #3). On the contrary, if the crystallization was directly performed without distillation of MeOH, it was confirmed that the loss by an aqueous layer was reduced (#4, #5, #6). However, even in this case, decreasing results of the yield were shown with the increase of the amount of water (#5, #6).

Accordingly, a secondary experiment on the crystallization conditions was performed as in Experimental Example 3.

### Experimental Example 3. Evaluation of crystallization conditions of 1-chloro-6-hydroxy-3,4-dihydronaphthalen-2-carbaldehyde 2

Through Experimental Example 2, even though the crystallization was performed using water and 3 N HCl without the distillation of methanol, there were problems in that a reactor wall was coated during crystallization according to the size of the reaction and the type of the reactor, or aggregation occurred to induce no precipitation.

Accordingly, the crystallization yield was evaluated by changing the dropwise addition rate of 3 N HCl, dropwise addition temperature or injection order during the crystallization, and the results are shown in Table 3.

**[Table 3]**

| Entr y | Cryst alliz ation order | Dropw ise addit ion temp (°C) | 3N HCl dropwi se additi on time | Agg reg ati on | Coating | | Note |
|---|---|---|---|---|---|---|---|
| | | | | | Reac tor wall | Stir rer | |
| 1 | 3N HCl → H₂O | < 10 | 2 h | Hig h | High | High | 1 L reactor test reaction |
| 2 | 3N HCl → H₂O | < 10 | 10 min | Mid dle | Low | Low | Black particles were coated to be converted into brown particles |
| 3 | 3N HCl → H₂O | < 10 | 2 h | Hig h | High | Midd le | Black particles were coated to be converted into brown particles |
| 4 | H₂O → 3N HCl | < 10 | 10 min | Low | Low | Low | No reactor wall coating phenomenon, slight aggregation during dropwise addition of H₂O |
| 5 | 3N HCl → H₂O | 30-35 | 10 min | Hig h | High | Midd le | Severe reactor wall coating |
| 6 | H₂O → | < 10 | 10 min | Low | Low | Low | Entry 4 scale up test; |
| | 3N HCl | | | | | | very slight coating on reactor wall and stirrer was observed, no other problems |
| 7 | 3N HCl → H₂O | < 10 | 10 min | Hig h | High | High | Black coating on the upper and lower reactor wall, brown coating on the middle of reactor wall; obtaining SG35 in a black oil type with poor crystallization |

From the results of Table 3, in the case of 3 N HCl injected during acidification, it was found that if the dropwise addition time increases, reactor wall coating and aggregation phenomenon became severe (#1, #2, #3). In addition, the high dropwise addition temperature also induced reactor wall coating problems. If the order of the dropwise addition was changed while maintaining a rapid dropwise addition rate to prevent the problems, it was observed that the coating phenomenon and aggregation phenomenon were solved (#4). It is thought that if H₂O was injected first, and then, acidification was performed, the K₂CO₃ remaining in the reaction solution was dissolved, and relatively uniform and stable crystallization was performed to relieve the aggregation and coating phenomena. As a result of reproduction experiment in a 35 g scale, the crystallization was performed without aggregation and coating problems, and discharge and filtering also were performed without problems. Accordingly, it was confirmed effective if the water was injected first and then, hydrochloric acid was injected during the crystallization.

### Experimental Example 4. Evaluation of crystallization solvent during synthesizing 1-chloro-6-(3-chloro-1-isopropyl-1H-indazol-5-ylmethoxy)-3,4-dihydro-naphthalen-2-carbaldehyde

During synthesizing 1-chloro-6-(3-chloro-1-isopropyl-1H-indazol-5-ylmethoxy)-3,4-dihydro-naphthalen-2-carbaldehyde (Example 3), a phenomenon of not separating and extracting the compound of Example 3 was observed through the simple injection of water after the coupling reaction of 5-bromomethyl-3-chloro-1-isopropyl-1H-indazole (Example 1) with 1-chloro-6-hydroxy-3,4-dihydronaphthalen-2-carbaldehyde (Example 2).

Accordingly, experiments on the crystallization conditions of 1-chloro-6-(3-chloro-1-isopropyl-1H-indazol-5-ylmethoxy)-3,4-dihydro-naphthalen-2-carbaldehyde was performed, and the results are shown in Table 4 and Table 5.

**[Table 4]**

| Entry | Conditions (fold) | N2 isome r (%) | Note |
|---|---|---|---|
| 1 | H₂O (14) add stirring → Dark solution, stick oil | - | Excessive amount of tar was observed; sticky oil and unable to crystallize |
| 2 | H₂O (10) add MTBE (5) add, cooling, stirring | - | Not crystallized |
| 3 | H₂O (10) add MTBE (5) + HEX (5) add, cooling, stirring | - | Not crystallized |
| 4 | EA extraction, NaOH (aq) washing DCM (3), MTBE (3), HEX (3) add | - | Not crystallized |
| 5 | EA extraction, NaOH (aq) | 3.6 | Good crystal |
| | washing EtOH (4), HEX (4) add | | formation |
| 5-1 | Entry 5 MTBE (3) washing | 3.4 | - |
| 6 | EA extraction, NaOH (aq) washing EtOH (5), HEX (4) add | 3.0 | - |
| 7 | H₂O (10) → EA (2), DCM (1) → clear → production of small amount of particles → Hex (15) | 0.8 | 62.3 % yield |
| 8 | H₂O (10) → EA (5) → clear → production of large amount of particles → H₂O (10) | 1.0 | 57.8 % yield |

As confirmed in Table 4, it was confirmed that crystal was not formed in some conditions, and it was confirmed that a low yield of 60% level was shown even in conditions of performing some crystallization. Accordingly, a secondary experiment was performed with the conditions of performing the crystallization.

**[Table 5]**

| Entr y | Conditions (fold) | Purity (%) | N2 isomer (%) | Net. Yield (%) |
|---|---|---|---|---|
| 1 | H₂O (10)/EtOH (2) mixture, dropwise | 88.9 | 3.0 | 87.4 |
| 2 | H₂O (10)/EtOH (1) mixture, dropwise | 89.5 | 2.9 | 91.2 |
| 3 | H₂O (10)/EtOH (4) mixture, dropwise | 91.0 | 2.8 | 93.4 |
| 4 | MTBE (2) → H₂O (10)/EtOH (2) mixture, dropwise | 91.4 | 2.2 | 90.7 |
| 5 | Hex (2) → H₂O (10)/EtOH (2) mixture, dropwise | 89.9 | 2.7 | 94.1 |
| 6 | H₂O (10)/EtOH (3)/EA (1) | 91.1 | 2.8 | 94.4 |
| 7 | H₂O (10)/EtOH (3)/DCM (1) | 90.4 | 2.7 | 94.6 |
| 8 | H₂O (10)/EtOH (3)/THF (1) | 89.7 | 2.9 | 85.7 |
| 9 | H₂O (10)/EtOH (3)/AN (1) | 90.0 | 2.9 | 93.2 |
| 10 | H₂O (10)/MeOH (4) | 89.9 | 2.9 | 94.7 |
| 11 | H₂O (10)/IPA (4) | 88.4 | 3.3 | 92.5 |
| 12 | H₂O (10)/EtOH (4) at 30°C | 90.4 | 2.8 | 82.9 |
| 13 | H₂O (10)/EtOH (4) for 2h | 91.2 | 2.7 | 90.1 |
| 14 | H₂O (10)/EtOH (4) poured | 90.1 | 2.8 | 93.8 |
| 15 | EtOH (4) → H₂O (10) for 2h | 91.1 | 2.6 | 90.8 |
| 16 | MTBE (2) → H₂O (10)/EtOH (4) for 2h | 95.6 | 1.4 | 82.5 |
| 17 | EA (2) → H₂O (10)/EtOH (4) for 2h | 91.7 | 2.7 | 92.9 |
| 18 | MTBE (2)/EtOH (2) for 1h → H₂O (10) for 1h | 92.3 | 2.2 | 91.9 |
| 19 | EA (2)/EtOH (2) for 1h → H₂O (10) for 1h | 90.4 | 3.0 | 91.8 |

Through the results of Table 4 and Table 5, it was confirmed that rapid aggregation phenomenon was observed in case of injecting only H₂O first, after performing the coupling reaction, and high reproduction and stable crystallization mode were shown in case of using the mixture solvent of H₂O/EtOH (10:4). Meanwhile, as in Table 4 and Table 5, if the mixture solvent of H₂O and EtOH was used, the crystallization with a yield of 87% or more could be achieved.

## Claims

1. A method for preparing an intermediate compound of Formula 4, the method comprising: a step of preparing an intermediate compound of Formula 4 by the coupling reaction of a compound of Formula 2 with a compound of Formula 3: 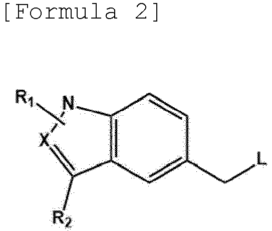 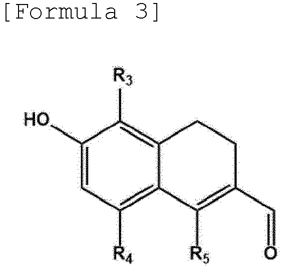 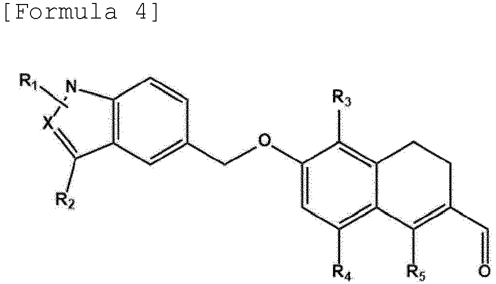 in the Formula 2 to the Formula 4,
R1 is hydrogen, or substituted or unsubstituted alkyl,
R2 is hydrogen, substituted or unsubstituted alkyl, halogen, CN, CF₃, or COCF₃,
R3 and R4 are each hydrogen, substituted or unsubstituted alkyl, or halogen,
R5 is hydrogen, substituted or unsubstituted alkyl, or halogen,
X is C or N, and
L is a leaving group.

2. The preparation method according to claim 1, wherein
R1 is C₁-C₄ substituted or unsubstituted alkyl,
R2 is halogen,
R3 and R4 are each hydrogen, or C₁-C₄ substituted or unsubstituted alkyl,
R5 is halogen,
X is N, and
L is the leaving group selected from chlorine (Cl), bromine (Br), iodine (I), methanesulfonate (Oms), p-toluenesulfonate (OTs), and trifluoromethanesulfonate (OTf).

3. The preparation method according to claim 1, wherein the compound of Formula 4 is obtained by including a step for crystallization, and
a solvent for crystallization comprises an alcohol-based solvent.

4. The preparation method according to claim 3, wherein the solvent for crystallization is a mixture solvent of the alcohol-based solvent and water.

5. The preparation method according to claim 1, wherein the compound of Formula 3 is prepared from a compound of the following Formula 5: 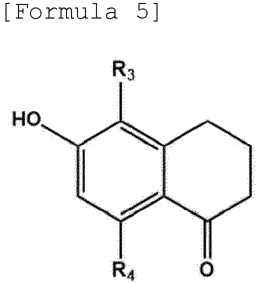 in the Formula 5, R3 and R4 are the same as defined in Claim 1.

6. The preparation method according to claim 5, wherein the compound of Formula 3 is prepared by the following steps:
1) a step of blocking an alcohol group of the compound of Formula 5 to prepare a compound of Formula 6;
2) a step of introducing an aldehyde into the compound of Formula 6 to prepare a compound of Formula 7; and
3) a step of restoring the alcohol group from the compound of Formula 7 to prepare the compound of Formula 3: 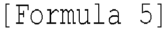 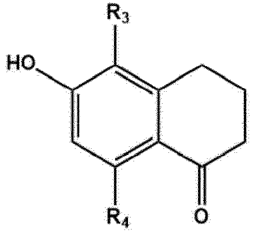 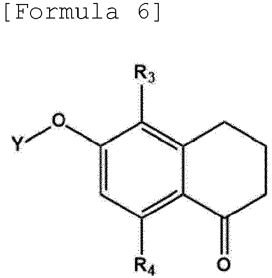 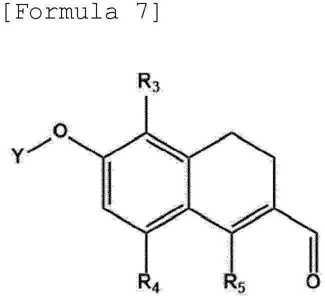
in the Formula 5 to the Formula 7,
each substituent is the same as defined in claim 1, and Y is an alcohol-protecting group.

7. The preparation method according to claim 5 or claim 6, wherein the compound of Formula 3 is obtained by including a step of crystallization under acid conditions.

8. The preparation method according to claim 7, wherein the step of crystallization is performed by injecting water and then injecting hydrochloric acid to a reaction product containing the compound of Formula 3.

9. The preparation method according to claim 5 or claim 6, wherein the compound of Formula 5 is prepared by dealkylation of a compound of the following Formula 8: 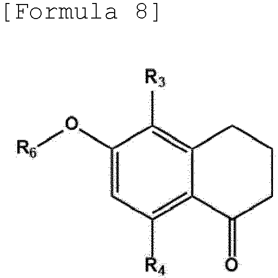 in the Formula 8,
R3 and R4 are the same as defined in claim 1, and
R6 is substituted or unsubstituted alkyl.
